# EUROPÄISCHE PATENTANMELDUNG

(11) **EP 0 627 232 A2**
(43) Veröffentlichungstag der Anmeldung: **07.12.1994**
(21) Anmeldenummer: 94104752.4
(22) Anmeldetag: 25.03.1994
(51) Int. Cl.: A61M 39/00, G02B 6/38

(54) **Steckverbindung**

(30) Priorität: 02.06.1993 DE 4318325
(71) Anmelder: DORNIER MEDIZINTECHNIK GMBH, D-82101 Germering (DE)
(72) Erfinder: Grözinger, Reiner, D-82239 Alling (DE); Buck, Roland, D-82110 Germering (DE); Buess, Gerhard, Prof.Dr., D-72074 Tübingen (DE)

(57) **Zusammenfassung**

Die Steckverbindung dient zum Anschluß mindestens einer sterilen elektrischen, optischen und/oder fluidführenden Leitung an mindestens ein unsteriles Gerät mit jeweils einem leitungsseitigen und einem geräteseitigen Steckerteil. Das geräteseitige Steckerteil ist mit mindestens einer sterilen Folie umgeben , die zwischen jeweils korrospondierende, die zu stekkende Leitung umgebende Dicht- und Klemmränder des leitungsseitigen und des geräteseitigen Steckerteils dicht eingeklemmt wird. Das leitungsseitige und/oder geräteseitige Steckerteil weist eine Trenn-oder Schneidvorrichtung zum Durchtrennen der Folie im Bereich der zu kontaktierenden Leitungen auf.

## Beschreibung

Die Erfindung betrifft eine Steckverbindung zum Anschluß mindestens einer sterilen elektrischen, optischen und/oder fluidführenden Leitung an mindestens ein unsteriles Gerät mit jeweils einem leitungsseitigen und einem geräteseitigen Steckerteil.

In den verschiedenen Anwendungsbereichen der Chirurgie ist es notwendig, innerhalb des sterilen OP-Bereiches Steckverbindungen zwischen Verbindungsleitungen vom Patienten zu technischen Versorgungseinrichtungen und umgekehrt herzustellen. Insbesondere bei der minimalinvasiven Chirurgie sind mehrere unterschiedliche Hilfsgeräte für die Präparation des OP-Feldes und den Chirurgieeingriff notwendig, bei denen Verbindungen mittels Schläuche und Kabel hergestellt werden müssen.

Bei einem in der älteren Anmeldung P 42 42 069.5 beschriebenen Arbeitsplatz-System wird zur Optimierung des Behandlungsablaufs die Zuführung der Verbindungsleitungen zum Patienten über ein zentrales Anschlußfeld durchgeführt, das sich innerhalb des sterilen OP-Bereiches befindet. Der Anschluß der Verbindungsleitungen am Anschlußfeld erfolgt zusammengefaßt mit einer oder mehreren Mehrfach-Steckverbindungen, wobei keine Berührung mit sterilen apparativen Komponenten im sterilen OP-Bereich und sterilem OP-Personal erfolgen darf. Dieses sterile Anschlußfeld wird zur Einhaltung der Sterilität vor dem Einschwenken in den sterilen OP-Bereich vom Hilfspersonal mit einer Sterilabdeckung (Schlauchbeutel) überzogen. Nach dem Einschwenken des Anschlußfeldes müssen die sterilen Verbindungsleitungen mit den sterilen Steckern unter Einhaltung der Sterilität durch steriles OP-Personal an dem zentralen Anschlußfeld konnektiert werden. Zur Konnektierung der Mehrfach-Steckverbindungen ist es notwendig, im Anschlußbereich aus der Sterilabdeckung passende Öffnungen herauszuschneiden.

Der Erfindung liegt die Aufgabe zugrunde, eine einfach zu handhabende Steckverbindung mit unterschiedlichen Typen von Verbindungsleitungen zu realisieren, mit dem diese Leitungen unter Sterilbedingungen innerhalb des sterilen OP-Bereiches an unsterile Geräte angeschlossen werden können, wobei während des Anschlußvorganges keinerlei unsterile Flächen freigelegt werden.

Diese Aufgabe erfüllt eine nach den Merkmalen des Patentanspruchs 1 ausgebildete Steckvorrichtung.

Mittels der Steckverbindung bleiben sterile und unsterile Teile insbesondere auch während des Steckvorganges hermetisch voneinander getrennt, wobei der Steckvorgang selbst leicht mit einer Hand durchzuführen ist. Hierzu ist der unsterile, geräteseitige Steckerteil einschließlich der daran befindlichen im sterilen OP-Bereich verlaufenden und zu kontaktierenden Leitungen (ggf. auch das Gerät selbst) von einer sterilen Folie umgeben. Das im sterilen OP-Bereich gelegene, mit zum Patienten führende Leitungen versehene Steckerteil bewirkt vor dem Einstecken in das geräteseitige Stekkerteil mit diesem eine Abdichtung des Kontaktbereiches durch Einklemmen der Folie zwischen zwei korrespondierende Dicht- und Klemmränder beider Steckerteile. Anschließend wird mittels einer Trenn-oder Schneidvorrichtung in der Folie eine Öffnung des Steckerbereiches für den Anschluß freigelegt. Die ausgeschnittene Folie wird in den Innenbereich des Steckers abgeführt, so daß anschließend die Verbindung zwischen den zu kontaktierenden Leitungen durchgeführt und die Steckverbindung als Ganzes arretiert werden kann.

Alle Funktionsteile für die Trenn- und Schneidvorrichtung sowie die einzelnen Steckerelemente der zu verbindenden Leitungen befinden sich innerhalb der geschlossenen, vorzugsweise kreisförmigen Dichtlippe, so daß nach der Penetration der Folie keine offene Verbindung von dem unsterilen geräteseitigen Steckerteil zum sterilen leitungsseitigen Steckerbereich besteht. Diese Dichtung schützt zusätzlich auch den inneren Steckerbereich vor Verschmutzungen von außen.

Die Erfindung wird im folgenden anhand eines in den Figuren teilweise schematisch dargestellten Ausführungsbeispieles näher beschrieben.

Es zeigen:
Fig. 1 a bis 1 d einen Querschnitt durch eine Steckverbindung in vier Phasen des Steckvorganges
Fig. 2 ein Anschlußfeld mit mehreren sterilen Steckverbindungen.

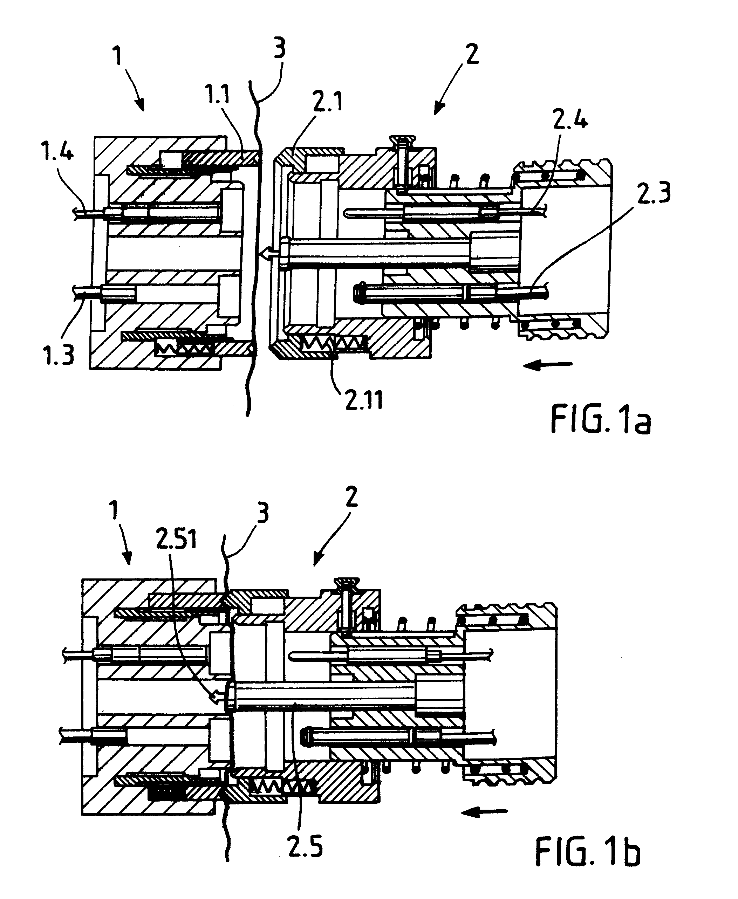

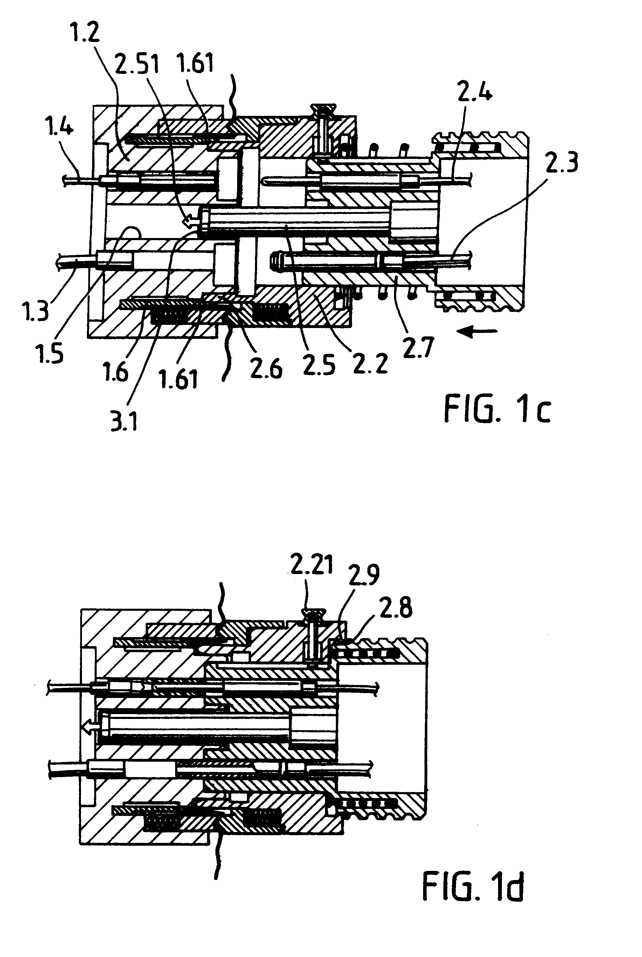

Das in den Figuren 1 bis d dargestellte Ausführungsbeispiel weist jeweils ein geräteseitiges unsteriles Steckerteil 1 sowie ein leitungsseitiges, steriles Steckerteil 2 auf. Beide Steckerteile sind vor dem Steckvorgang durch eine sterile Folie 3 voneinander getrennt, wobei die Folie Im weiteren und nicht dargestellten Bereich den in den OP hineinragenden unsterilen Geräteteil von dem sterilen Bereich des OP-Feldes trennt. Beide Steckerteile 1 und 2 weisen jeweils aufeinanderpassende, federnd gelagerte Dicht- und Klemmringe 1.1 und 2.1 auf, welche das Kontaktfeld der zu kontaktierenden Leitungen 1.3, 1.4 bzw. 2.3, 2.4 umgeben.

Im dargestellten Ausführungsbeispiel handelt es sich bei der zu steckenden Leitung 1.3 und 2.3 um eine fluidführende Leitung, z.B. um eine mit einer Vakuumpumpe verbundene Absaugleitung und bei der Leitung 1.4 bzw. 2.4 um eine isolierte elektrische Kontaktleitung. Ob und in welcher Weise solche unterschiedlichen Leitungsarten in einem Stekker kombiniert werden sowie deren Anzahl, hängt jedoch insbesondere von sicherheitstechnischen Überlegungen ab.

Mit der ersten, in Fig. 1 gezeigten Berührung der beiden Steckerteile 1 und 2 wird die Folie 3 zwischen den Dicht- und Klemmringen 1.1 und 2.1 eingeklemmt sowie von einem Dornfortsatz 2.51, der an der Spitze eines Stößels 2.5 im Zentrum des rechten Steckerteiles angeordnet ist, perforiert. Die so eingeklemmte und gespannte Folie 3 wird bei weiterem Einschieben des rechten Steckerteiles 2 durch einen konzentrisch zum Dicht- und Klemmring 2.1 verlaufenden Ring 2.6 gespannt und an ein konzentrisch zum Dicht- und Klemmring 1.1 verlaufenden Schneidring 1.6 gedrückt, welcher mit Messern 1.61 besetzt ist (siehe Figur 1 c). Bei Erreichen einer vorbestimmten Position des einfedernden Dicht- und Klemmringes 1.1 löst dieser über einen nicht dargestellten Schalter einen , ebenfalls nicht dargestellten elektrischen Antrieb für den Schneidring 1.6 aus, wodurch dieser in Drehung versetzt wird und aus der Folie 3 den innerhalb des Dicht- und Klemmränder 1.1 und 2.1 gelegenen Bereich herausschneidet. Das herausgeschnittene Folienstück 3.1 wird durch den Stößel 2.5 in eine zentrale Bohrung 1.5 des Steckerteiles 1 hineingedrückt und somit aus dem eigentlichen Kontaktfeld herausgezogen. In dieser Phase des Steckvorganges sitzt das Mittelteil 2.2 des Steckerteiles 2 bündig und eindeutig positioniert am Mittelstück 1.2 des Steckerteiles 1. Das die zu steckenden Kontakte 2.3 und 2.4 tragende Steckerelement 2.7 des Steckerteiles 2 ist in einer Führung des Steckerelementes 2.2 axial verschiebbar und wird im weiteren Verlauf des Steckvorganges gegen das Mittelstück 1.2 des Steckerteiles 1 geschoben, wodurch die zu steckenden Leitungen in Verbindung treten. Am Ende des Steckvorganges rastet eine Arretierung 2.8 am Steckerelement 2.7 in eine korrespondierende Nut 2.9 des Steckerelementes 2.2, welches seinerseits mit dem geräteseitigen Steckerteil 1 bzw. 1.2 durch eine Bajonettverriegelung fest verbunden ist.

Zum Lösen der Steckverbindung wird die Arretierung 2.8 gelöst, daraufhin das Steckerelement 2.7 zurückgezogen und schließlich durch Drehung die Bajonettverriegelung des Steckerelementes 2.2 vom Steckerteil 1 gelöst. Gleichzeitig wird damit das am Dornfortsatz 2.51 hängende Folienstück 3.1 aus der Öffnung 1.5 herauszogen und kann somit entfernt werden.

Zum Zwecke der vollständigen Zerlegung und Sterilisierung kann das Steckerteil 2 weiter in die Elemente 2.2 und 2.7 zerlegt werden, in dem die Verriegelung und Verdrehsicherung 2.21 angehoben und das Steckerelement 2.7 aus der Führung des Steckerelementes 2.2 herausgezogen wird. Weiterhin kann der mittels der Feder 2.11 gehaltene Dicht- und Klemmring 2.1 abgezogen werden.

Die Befestigung der Steckkontakte 1.3, 1.4 bzw. 2.3, 2.4 in den jeweiligen Steckerelementen 1 und 2 ist so gestaltet, daß bei Störungen die einzelnen Verbindungen unter Sterilbedingung gewechselt werden können, in dem diese jeweils auf der Steckerrückseite abgezogen und durch neue Steckkontakte ersetzt werden; solange ein solcher Kontaktwechsel nicht gleichzeitig auf beiden Stekkerteilen 1 und 2 geschieht, bleibt die Trennung zwischen unsterilem und sterilem Bereich gewahrt.

Anstelle des mit Messern 1.61 besetzten Schneidringes 1.6 kann alternativ auch ein kreisförmiger Heizdraht installiert sein, der durch kurzzeitiges Erhitzen während des Steckvorganges die Folie aus dem Kontaktbereich herausschneidet.

Anstelle des in dem Ausführungsbeispiel gezeigten Stößels 2.5 kann die Folie auch durch eine Saugeinrichtung über den Kanal 1.5 abgesaugt werden. Als Folie eignet sich insbesondere ein transparentes dehnbares Material mit einer Dicke zwischen 50 und 100 u.

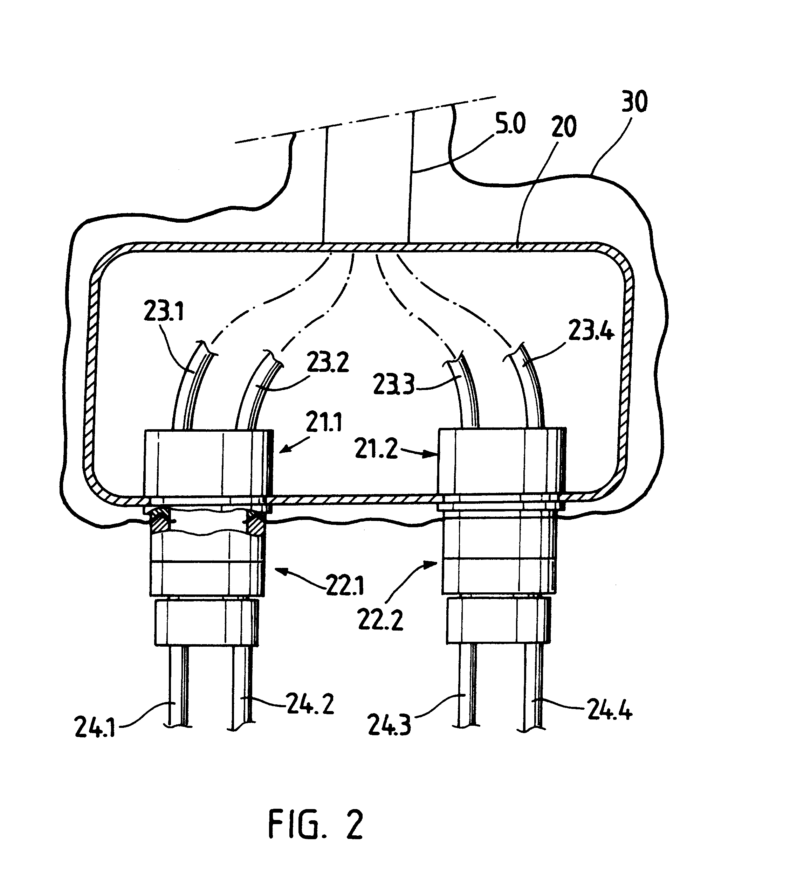

Bei dem in Fig. 2 dargestellten Ausführungsbeispiel sind zwei geräteseitige Steckerteile 21.1 und 21.2 nach Art der Ausführung gemäß Fig. 1 auf einem Anschlußfeld 20 angeordnet, wobei die jeweils zu steckenden Leitungen 23.1 bis 23.4 über einen Schacht 50 zu den im unsterilen Bereich befindlichen Geräten geführt werden. Das gesamte Anschlußfeld einschließlich des Schachtes 50 ist von einem Foliensack 30 umgeben, der die Trennung zwischen sterilem und unsterilem Bereich dargestellt.

An die im Anschlußfeld angeordneten Steckerteile 21.1 und 21.2 sind korrespondierende Stekkerteile 22.1 bzw. 22.2 in der, unter Figur 1 beschriebenen Ausführung anschließbar. Die an diesen Steckerteilen 22.1 und 22.2 angeordneten Leitungen 24.1, 24.2, 24.3 und 24.4 führen zum sterilen Operationsfeld.

## Patentansprüche

1. Steckverbindung zum Anschluß mindestens einer sterilen elektrischen, optischen und/oder fluidführenden Leitung an mindestens ein unsteriles Gerät mit jeweils einem leitungsseitigen und einem geräteseitigen Steckerteil, dadurch gekennzeichnet, daß das geräteseitige Steckerteil (1) mit mindestens einer sterilen Folie (3) umgeben ist, daß das leitungsseitige und das geräteseitige Steckerteil (2, 1) jeweils korrospondierende, die zu steckende Leitung (1.3, 1.4; 2.3, 2.4) umgebende Dicht- und Klemmränder (1.1; 2.1) für die Folie (3) aufweisen und, daß das leitungsseitige und/oder geräteseitige Steckerteil (2, 1) eine Trenn- oder Schneidvorrichtung (1.6) zum Durchtrennen der Folie (3) im Bereich der zu kontaktierenden Leitungen (1.3, 1.4; 2.3, 2.4) aufweist.

2. Steckerverbindung nach Anspruch 1, dadurch gekennzeichnet, daß das leitungs- und/oder geräteseitige Steckerteil (1, 2) einen federnd gelagerten Ring (1.1; 2.1) mit einem Dicht- und Klemmrand aufweist.

3. Steckverbindung nach Anspruch 1 oder 2, dadurch gekennzeichnet, daß mittels der Trenn- oder Schneidvorrichtung (1.6) ein Auschnitt aus der Folie (3) im Bereich der zu kontaktierenden Leitungen (1.3, 1.4; 2.3, 2.4) herstellbar ist.

4. Steckverbindung nach einem der Ansprüche 1 bis 3, dadurch gekennzeichnet, daß die Trenn- oder Schneidvorrichtung (1.6) innerhalb eines Steckerteils (1) angeordnet ist und Messer aufweist, welche auf der Innenseite des Dicht- und Klemmrandes (1.1) kreisbogenförmig bewegbar sind.

5. Schneidvorrichtung nach Anspruch 4, dadurch gekennzeichnet, daß die Messer (1.6) einen durch die Steckverbindung auslösbaren Antrieb aufweisen.

6. Steckverbindung nach einem der Ansprüche 1 bis 5, dadurch gekennzeichnet, daß die Trenn- oder Schneidvorrichtung einen entlang der Innenseite des Dicht- und Klemmrandes verlaufenden Heizdraht aufweist.

7. Steckverbindung nach einem der Ansprüche 1 bis 6, dadurch gekennzeichnet, daß die Trenn- oder Schneidvorrichtung eine entlang der Innenseite des Dicht- und Klemmrandes verlaufende Stanzvorrichtung aufweist.

8. Steckverbindung nach einem der Ansprüche 1 bis 7, gekennzeichnet durch eine Saug-, Blas- oder Stoßvorrichtung (2.5) zum Entfernen des Folienausschnittes aus der Kontaktebene der Steckerteile (1, 2) vor Kontaktierung der Leitungen (1.3, 1.4; 2.3, 2.4) .

9. Steckverbindung nach einem der Ansprüche 1 bis 8, dadurch gekennzeichnet, daß das leitungsseitige Steckerteil (2) ein mit zu steckenden Leitungen (2.3, 2.4) versehenes Innenteil (2.7) und ein, diesem gegenüber axial verschiebbares Koppelteil (2.2) mit einem Dicht-und Klemmrand (2.1) sowie Kupplungselementen aufweist.

10. Steckverbindung nach einem der Ansprüche 1 bis 10, dadurch gekennzeichnet, daß die sterile Folie (3) eine Dicke zwischen 50 11. und 100 µ, aufweist und aus einem glatten, dehnbaren Material besteht.

11. Steckverbindung nach einem der Ansprüche 1 bis 11, dadurch gekennzeichnet, daß mehrere geräteseitige Steckerteile (21.1, 21.2) auf einem Anschlußfeld (20) angeordnet und von einer geschlossenen Folie (30) umgeben sind.

12. Steckverbindung nach einem der Ansprüche 1 bis 11, dadurch gekennzeichnet, daß die zu kontaktierenden Leitungen (1.3, 1.4; 2.3, 2.4) aus den gekoppelten Steckerteilen (1, 2) herausziehbar sind.
